Europäisches Patentamt

European Patent Office

Office européen des brevets

(19)

(11) Publication number: 0 339 011
A1

# EUROPEAN PATENT APPLICATION

(12)

(21) Application number: 89870053.9

(51) Int. Cl.⁴: C 12 N 9/18

(22) Date of filing: 17.04.89

(30) Priority: 19.04.88 LU 87203

(43) Date of publication of application:
25.10.89 Bulletin 89/43

(84) Designated Contracting States: ES GR

(71) Applicant: RIJKSUNIVERSITEIT GENT
Sint-Pietersnieuwstraat 25
B-9000 Gent (BE)

(72) Inventor: Vandamme, Erick-Jérome-Maurice-Cornelius
Katwilgenstraat 10
B-9030 Gent/Wondelgem (BE)

Vermeire, Annie Maria Magdaléna
Riemsesteenweg, 136
B-9068 Ertvelde (BE)

(74) Representative: Van Malderen, Michel et al
p.a. Office van Malderen avenue J.S. Bach 22/43
B-1080 Bruxelles (BE)

(54) Process for the preparation of tannase intended for the production of gallic acid, with the aid of an aspergillus culture.

(57) Production of tannase characterized in that the latter is prepared by fermentation of Aspergillus tamarii, in particular the CBS 10413 strain.
  Tannase is obtained with a high production efficiency and is employed for the production of gallic acid.

EP 0 339 011 A1

## Description

## Process for the preparation of tannase intended for the production of gallic acid, with the aid of an Aspergillus culture.

### SUBJECT OF THE INVENTION

The present invention relates to a process for the preparation of a tannase from microorganisms, mainly by means of a particular strain of Aspergillus.

The invention extends to the tannase prepared by means of this process and to the use of this tannase for the preparation of gallic acid.

### SUMMARY OF THE STATE OF THE ART

Tannase, an enzyme discovered by Scheele in 1786, exhibits the property of hydrolysing the ester bond as well as the depside bond of tannic acid and glucose.

The preparation of tannase from various types of Aspergillus or Penicillium has already been described.
- Freudenberg et al. (Z.Physiol.Chem. 164, 262, 1927); have prepared tannase from Aspergillus niger, and
- K. Yamada et al. (J. Ferment. Tech., 45, 233, 1967); (Agr. Biol. Chem. 31, 513, 1967) have prepared tannase from Aspergillus oryzae.

The use of Aspergillus oryzae is also described in Chemical Abstracts, vol.83, No. 9, 1 September 1975, page 508, abstract no. 76990g, Columbus, Ohio, US; and JP-A-75/25,786 (Kikkoman Shoyu Co., Ltd) 18-03-1975.

Document GB-A-1,249,932 (Tenco Brooke Bond Ltd) describes the use of a tannase derived from Aspergillus, niger, oryzae or flavus for the treatment of "tea cream".

### CHARACTERISTIC FEATURES OF THE INVENTION

The Applicant Company has found that, surprisingly, the genus Aspergillus tamarii, in particular the CBS 10413 strain, is characterized by a very highly efficient production of tannase. The said strain is characterized by a high specificity together with a high volumetric activity in respect of tannase during the fermentation process.

According to the invention, tannase is obtained by fermentation of Aspergillus tamarii in a medium containing tannins intended to induce the synthesis of tannase. The fungal mycelium can be isolated from the fermentation medium and employed repeatedly in the form of a tannase enzyme system immobilized on a support, in situ, for the bioconversion of tannins. The biomass can be recycled and reused without addition of any special nutrient agent. The gallic acid obtained has the conventional properties of commercial gallic acids and is in the form of a practically colourless crystalline powder which is soluble in water, in ethanol and in acetone. This compound is employed, inter alia, for the preparation of gallic esters for use in foodstuffs and in pharmaceutical compounds.

The starting tannins form a complex mixture of polyphenolic compounds which are present in many plants. They are categorized into condensed tannins and into hydrolysable tannins. Gallotannins are hydrolysable either by an enzyme route or by a chemical route, producing gallic acid and releasing the central molecule of the nucleus (consisting of glucose or quinic acid).

It is appropriate to note that it is already known and proven that fermentation processes can succeed or fail, depending on the suitable or unsuitable choice of an appropriate strain. The Applicant Company has noticed, in particular, that among the many cultures selected and isolated on agar culture plates and having received a preparation of tannins, very few compounds exhibit a highly efficient production of tannase in a liquid culture medium.

The particular choice of the Aspergillus tamarii species for the preparation of tannase makes it possible, surprisingly, to obtain tannase production efficiency characteristics which are superior to the result obtained according to the state of the art.

### PREFERRED EMBODIMENTS OF THE INVENTION

The invention will be described in greater detail with the aid of specific examples of the uses of individual strains according to the invention of the Aspergillus genus.

#### Measurement of the activity in respect of tannase

The activity in respect of tannase can be measured by various techniques which have already been described in literature. A particular technique has been developed by Iibuchi et al. (Agr.Biol.Chem. 31, 513, 1967). To do this, the hydrolysis of a standardized solution of tannins is followed by spectrophotometry and is directly related to the activity in respect of tannase. The activity in respect of tannase is expressed in terms of a tannase unit (tannase units = TU). A TU unit is the quantity of tannase which hydrolyses one micromole of ester bonds of gallic acid in one minute after incubation under optimum reaction conditions. As described initially in the above reference, this technique has been slightly modified as follows:

1 gram of fungal mycelium is incubated in a 1-2% strength solution of tara-tannin at pH 5.5 (0.1 M citrate buffer) for 30 minutes at 30°C. Before and after incubation, 50 µl of the tannin solution are transferred with a pipette into 10 ml of 90% ethanol. The drop in the absorption is measured at 310 nm.

The enzyme activity can be calculated from a purified tannase standard used as reference. The reference tannase activity is determined according to Iibuchi et al.

Process for the fermentation of Aspergillus tamarii CBS 10413 with a view to the production of tannase.

Since the biosynthesis of tannase constitutes an operation which must be induced, it is appropriate to add tannins to the fermentation media. An addition of tannins to the preculture as well as to the germination medium is recommended in order to obtain fast subsequent growth and production stages. The concentration of tannins in the fermentation liquid can vary between 1 and 30% (weight/volume), although the germination and preculture media must contain only low levels of tannins (1 to 5% - weight/volume) for the induction.

In the absence of additional carbon sources, the compounds resulting from the hydrolysis of the tannin (gallic acid, glucose and quininic acid) can be metabolized by the mycelium. The fermentation operation can be accelerated by the addition of sugars to the fermentation media. Sucrose, glucose, fructose and its industrial sources, namely molasses and glucose and fructose syrups can be added in concentrations of the order to 2 to 4% (weight/volume) depending on the level of the nitrogen which is fed. A high C/N ratio (of the order of 10 to 15) promotes the production of a highly active biomass. The nitrogen can be fed in the form of inorganic ammonium and nitrate salts such as: $(NH_4)_2SO_4$, $(NH_4)_2CO_3$, $(NH_4)_2HPO_4$, $NH_4Cl$, $NH_4NO_3$, $KNO_3$ and $NaNO_3$.

Organic nitrogenous sources (beef extract, soya peptone, peptone, slop, dried soluble distillation residues, molasses extract liquor, blood flour, soya flour) cannot be employed because, in the case of all these compounds, a precipitation reaction is observed with tannins.

Aspergillus tamarii, in particular the CBS 10413 strain, is relatively undemanding in respect of minerals. K, Mg, Zn, P and S are added to the culture medium in the form of inorganic sulphate or phosphate salts. Folic acid and pantothenic acid may be added as additional growth agents.

EXAMPLES

The invention will be described in greater detail with reference to two particular examples of implementation of the invention:

Example 1. Tannase fermentation starting with Aspergillus tamarii CBS 10413

Composition of the medium (g/l)

| Tannins | 10 to 300 |
|---|---|
| Sucrose | 30 |
| $KNO_3$ | 8.5 |
| KCl | 1.0 |
| $(NH_4)_2HPO_4$ | 1.0 |
| $MgSO_4.7H_2O$ | 0.5 |
| $ZnSO_4.7H_2O$ | 0.3 |
| Folic acid | 1 mg/l |
| Pantothenic acid | 1mg/l |

Tannase-producing fermentation starting with Aspergillus tamarii CBS 10413 has been investigated in a conventional stirred reactor (Applikon, Biolaffite).

The production of tannase is carried out at 30°C. During the fermentation (in particular during the growth and exponential production stage), the pH changes rapidly. NaOH (5 N) and $H_2SO_4$ (5 N) are added during the fermentation process in order to maintain the pH between 3.5 and 4.5.

The fermentation liquid is aerated by blowing in 0.5 to 1 vvm of oxygen-saturated air into the culture medium. The rate of agitation is adjusted to the rate of oxygen transfer. The reactor (10 l) is inoculated with 10% (v/v) of preculture or of germination medium, which, in its turn, is prepared from a fresh spore suspension (5% volume/volume) collected from a malted agar medium. The composition of the liquid medium is identical for the various fermentation steps. At the end of the fermentation (exhaustion of the carbon substrate) the biomass is separated from the fermentation broth by vacuum filtration. The biomass is washed and kept at -18°C. An intercellular tannase activity of 15,000 units/l can be obtained. Approximately 3,000 units/l are secreted into the fermentation broth.

Example 2. Sequence for producing tannase from Aspergillus tamarii CBS 10413

The preparative sequence is illustrated in table I, appended.

The inoculum is prepared as follows: spores originating from an initial spore culture, which have been grown on agar/malt extract (Roux flasks) are transferred into a physiological saline solution (150ml). The germination medium (g/l) made up as follows:

| | |
|---|---|
| tannins | 50 |
| glucose | 30 |
| KNO$_3$ | 8.5 |
| K$_2$HPO$_4$ | 1.0 |
| MgSO$_4$.7H$_2$O | 0.5 |
| ZnSO$_4$.7H$_2$O | 0.5 |
| NaCl | 1.0 |

is inoculated with a 10% (volume/volume) suspension of the said spores. The germination medium is adjusted to pH 5 before sterilisation. After 16 to 24 hours' incubation of the culture in flasks on a mechanical shaker at 30°C, the germination medium is transferred into the agitated reactor containing 5 l of preculture (at a concentration of 10% volume/volume), the total volume of the reactor being 10 l. The preculture medium (g/l) is made up as follows:

| | |
|---|---|
| tannins | 50 |
| glucose | 30 |
| NH$_4$Cl | 3.75 |
| KCl | 1.0 |
| (NH$_4$)$_2$HPO$_4$ | 1.0 |
| MgSO$_4$.7H$_2$O | 0.5 |
| ZnSO$_4$.7H$_2$O | 0.5 |
| NaCl | 1.0 |
| Folic acid | 1 mg/l |
| pantothenic acid | 1 mg/l |

The fermentation reactor (containing the medium) is sterilized at 121°C for 30 minutes. Tannin is added to the medium after sterilization to avoid chemical hydrolysis of the induction molecules. After inoculation, the preculture is incubated for 48 h at 30°C. An aeration of 1 vvm and an agitation of 400 rev/min are maintained. Variations in the pH values of the fermentation medium of between 3.0 and 4.5 are permitted. Adjustments of pH are performed using sulphuric acid and sodium hydroxide.

After 36 hours' incubation, the preculture is transferred into a 100-litre fermenter (70 l working capacity). The composition of the medium, as well as the preparation and the sterilization, are identical with those shown for the preculture.

The fermentation is conducted at 30°C, 1 vvm, 300 rev/min and a pH of 3.0 to 4.5.

Frothing is controlled by adding a silicone-based foam suppressor. After 38 h of incubation the fermentation is stopped; all the available carbon sources have been metabolized. The fermentation medium is filtered and washed with demineralized water. The biomass (1.125 kg of dried cell mass) is stored at -18°C. The total intercellular activity was 1.125 x 10$^6$ units. The extracellular activity was approximately 1.5 x 10$^5$ or 12% of the total volumetric activity.

Table 1 (Example 2)

Agar + malt extract

$30^{\circ}$C, 3 days

Aspergillus tamarii CBS 10413 spores

physiological saline solution (0.75% NaCl)
spore suspension

Germination medium                     (spore suspension 5%)

Preculture - 10 l

Fermenter - 100 l

Filtration

Filtrate

Biomass - tannase

**Claims**

1. Process for the preparation of tannase, characterized in that the genus Aspergillus tamarii, in particular the CBS 10413 strain, is employed for the production.

2. Process according to Claim 1, characterized in that the tannase is obtained by fermentation of Aspergillus tamarii in a medium containing tannins intended to induce the synthesis of tannase, it being possible for the fungal mycelium to be isolated from the fermentation medium and employed repeatedly in the form of a tannase enzyme system immobilized on a support, in situ, for the bioconversion of tannins and it being possible for the biomass to be recycled and reused without addition of any special nutrient agent.

3. Process according to Claim 2, characterized in that tannins are added to the preculture and to the germination medium to obtain fast subsequent growth and production stages.

4. Process according to Claim 3, characterized in that the concentration of tannins in the fermentation liquid is between 1 and 30% (weight/volume), while the germination and preculture media contain only low levels of tannins (1 to 5% weight/volume) for the induction.

5. Process according to any one of Claims 1 to 4, characterized in that the fermentation operation is accelerated by the addition of sugars to the fermentation media.

6. Process according to Claim 5, characterized in that sucrose, glucose, fructose or their industrial sources, namely molasses and glucose and fructose syrups, are added in concentrations of the order of 2 to 4% (weight/volume) depending on the level of nitrogen which is fed.

7. Process according to Claim 4 or 5, characterized in that a high C/N ratio (of the order of 10 to 15) is produced to promote the production of a highly active biomass.

8. Process according to Claim 7, characterized in that the nitrogen is fed in the form of inorganic ammonium and nitrate salts such as : $(NH_4)_2SO_4$, $(NH_4)_2CO_3$, $(NH_4)_2HPO_4$, $NH_4Cl$, $NH_4NO_3$, $KNO_3$ and $NaNO_3$.

9. Process according to any one of Claims 2 to 8, characterized in that K, Mg, Zn, P and S minerals are added to the culture medium in the form of sulphate or inorganic phosphate salts.

10. Process according to any one of Claims 2 to 9, characterized in that folic acid and pantothenic acid are added as additional growth agents.

11. Tannase obtained by the process of any one of Claims 1 to 10.

12. Use of tannase according to Claim 11 for the production of gallic acid.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | GB-A-1 249 932 (TENCO BROOKE BOND) * Claims * --- | 1 | C 12 N 9/18 |
| A | CHEMICAL ABSTRACTS, vol. 83, no. 9, 1st September 1975, page 508, abstract no. 76990g, Columbus, Ohio, US; & JP-A-75 25 786 (KIKKOMAN SHOYU CO., LTD) 18-03-1975 (Cat. D) ----- | 1 | |

TECHNICAL FIELDS SEARCHED (Int. Cl.4)

C 12 N

The present search report has been drawn up for all claims

| Place of search | Date of completion of the search | Examiner |
|---|---|---|
| THE HAGUE | 18-07-1989 | DELANGHE L.L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                

& : member of the same patent family, corresponding document

EPO FORM 1503 03.82 (P0401)